(19) Europäisches Patentamt
European Patent Office
Office européen des brevets

(11) EP 1 441 714 B1

(12) **EUROPEAN PATENT SPECIFICATION**

(45) Date of publication and mention of the grant of the patent:
**26.12.2007 Bulletin 2007/52**

(21) Application number: **02787507.9**

(22) Date of filing: **24.10.2002**

(51) Int Cl.:
***A61K 31/196*** *(2006.01)* ***A61P 35/00*** *(2006.01)*
***A61K 31/502*** *(2006.01)* ***A61K 31/195*** *(2006.01)*
***A61K 31/50*** *(2006.01)*

(86) International application number:
**PCT/EP2002/011924**

(87) International publication number:
**WO 2003/035047 (01.05.2003 Gazette 2003/18)**

(54) **COMBINATIONS COMPRISING A SELECTIVE CYCLOOXYGENASE-2 INHIBITOR**
KOMBINATIONSMEDIKATIONEN MIT EINEM SELEKTIVEN CYCLOOXYGENASE-2-INHIBITOR
COMBINAISONS COMPRENANT UN INHIBITEUR SELECTIF DE LA CYCLOOXYGENASE-2

(84) Designated Contracting States:
**AT BE BG CH CY CZ DE DK EE ES FI FR GB GR IE IT LI LU MC NL PT SE SK TR**
Designated Extension States:
**SI**

(30) Priority: **25.10.2001 US 344734 P**
**25.10.2001 US 344735 P**
**15.11.2001 US 336033 P**

(43) Date of publication of application:
**04.08.2004 Bulletin 2004/32**

(73) Proprietors:
- **Novartis AG**
**4056 Basel (CH)**
Designated Contracting States:
**BE BG CH CY CZ DE DK EE ES FI FR GB GR IE IT LI LU MC NL PT SE SK TR**
- **Novartis Pharma GmbH**
**1235 Vienna (AT)**
Designated Contracting States:
**AT**

(72) Inventors:
- **CHEN, Ying-Nan, Pan**
**Parsippany, NJ 07054 (US)**
- **LASSOTA, Peter**
**Succasunna, NJ 07876 (US)**
- **WOOD, Alexander, Wallace**
**Ho Ho Kus, NJ 07423 (US)**

(74) Representative: **de Weerd, Petrus G.W. et al**
**Novartis International AG**
**Corporate Intellectual Property**
**4002 Basel (CH)**

(56) References cited:
**EP-A- 0 927 555**
**WO-A-01/12227**
**WO-A-01/40216**
**WO-A-01/47507**
**WO-A-01/60365**
**WO-A-01/62252**
**WO-A-01/64669**
**WO-A-98/35958**
**WO-A-99/11605**
**US-A- 6 034 256**

- **PROC AM SOC CLIN ONCOL, ABSTR 270, vol. 20, 2001,**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 441 714 B1

## Description

The invention relates to preventing or treating pre-malignant colon lesions (e.g. polyps) and colon cancer, as well as other malignancies in a warm-blooded animal, especially a mammal, particularly a human, with a combination of pharmaceutical agents which comprises (a) a selective cyclooxygenase-2 inhibitor ("COX-2 inhibitor") as defined in the claims and (b) an epothilone. The invention further relates to pharmaceutical compositions comprising (a) said COX-2 inhibitor and (b) an epothilone, and (c) a pharmaceutically acceptable carrier. The present invention further relates to a commercial package or product comprising (a) a pharmaceutical formulation of said COX-2 inhibitor and (b) an epothilone for simultaneous, concurrent, separate or sequential use.

WO9911605A1 discloses certain 5-ALKYL-2-ARYLAMINOPHENYLACETIC ACIDS AND DERIVATIVES thereof which are particularly potent and selective cyclooxygenase-2 (COX-2) inhibitors, methods for preparation thereof, pharmaceutical compositions comprising said compounds, methods of selectively inhibiting COX-2 activity and of treating conditions in mammals which are responsive to COX-2 inhibition using said compounds or pharmaceutical compositions comprising said compounds of the invention.

WO0160365A1 discloses the treatment or prevention of prostate cancer using cyclooxygenase-2 (COX-2) selective inhibiting drugs.

EP-A-0927555 discloses the use of certain compounds, specifically cyclooxygenase-2 inhibitors ("COX-2 inhibitors") for the treatment and prevention of tumours and tumour-related disorders and cachexia.

US-A-6034256 is in the field of anti-inflammatory pharmaceutical agents and specifically relates to compounds, compositions and methods for treating cyclooxygenase-2 mediated disorders, such as inflammation and inflammation-related disorders.

The two following abstracts

> **"Phase I Evaluation of an Epothilone B Analog (BMS-247550): Clinical Findings and Molecular Correlates. *S Mani, H McDaid, H Shen, JA Sparano, A Hamilton, C Runowicz, H Hochster, F Muggia, A Fields, B Damle, S Letrent, D Lebwohl, SB Horwitz (ABSTRACT 269)*.**" and
> **"A Phase I and Pharmacologic Trial of Weekly Epothilone B in Patients with Advanced Malignancies. *EH Rubin, LL Siu, S Beers, MJ Moore, C Thompson, M Becker, TL Chen, P Cohen, J Rothermel, AM Oza (ABSTRACT 270)*."** disclose that

Epothilone A and B are natural marine products and BMS-247550 is an Epothilone B analogue. All three molecules demonstrate broad antitumor activity, bind tubulin, and demonstrate activity in taxane-resistant tumors in vitro. They also demonstrate activity in tumor cell lines that overexpress the transmembrane drug export protein MDR-1, suggesting that the epothilones are not substrates for MDR-1. *Drs. Mani* and *Rubin* disclose phase I trials, reviewing the safety, maximum tolerated dose, and preliminary clinical activity of these molecules. Dose-limiting toxicity with the current schedules was diarrhea. Responses were seen in both trials in women with taxane-treated ovarian cancer. Phase II trials in ovarian carcinoma are under way with both of these interesting compounds.

Non-steroidal antiinflammatory agents are known to block prostaglandin synthesis by inhibition of the enzyme cyclooxygenase. Cyclooxygenase is now known to comprise a constitutive isoform, cyclooxygenase-1 ("COX-1"), and an inducible isoform, cyclooxygenase-2 ("COX-2").

COX-2 inhibitors are known in the art as compounds that selectively inhibit cyclooxygenase-2 without appreciable inhibition of cyclooxygenase-1. Methods of measuring the inhibition of cyclooxygenase-1 and -2 are known in the art.

Of the known COX-2 inhibitors, the 5-alkyl substituted 2-arylaminophenylacetic acids and derivatives are especially useful in the present invention. Such compounds, their use, preparation and galenical formulations comprising such compounds are disclosed in U.S. Patent No. 6,291,523.

Useful COX-2 inhibitors disclosed in U.S. Patent No. 6,291,523 are described by formula I

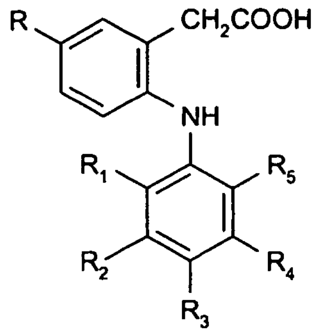

(I)

wherein R is methyl or ethyl;
$R_1$ is chloro or fluoro;
$R_2$ is hydrogen or fluoro;
$R_3$ is hydrogen, fluoro, chloro, methyl, ethyl, methoxy, ethoxy or hydroxy;
$R_4$ is hydrogen or fluoro; and
$R_5$ is chloro, fluoro, trifluoromethyl or methyl;
pharmaceutically acceptable salts or solvates thereof; and
pharmaceutically acceptable prodrug esters thereof.

A particular embodiment of the invention relates to the compounds of formula I wherein R is methyl or ethyl; $R_1$ is chloro or fluoro; $R_2$ is hydrogen; $R_3$ is hydrogen, fluoro, chloro, methyl or hydroxy; $R_4$ is hydrogen; and $R_5$ is chloro, fluoro or methyl; pharmaceutically acceptable salts thereof.

A preferred embodiment relates to the compounds of formula I wherein R is methyl or ethyl; $R_1$ is fluoro; $R_2$ is hydrogen; $R_3$ is hydrogen, fluoro or hydroxy; $R_4$ is hydrogen; and $R_5$ is chloro; pharmaceutically acceptable salts thereof.

Another preferred embodiment of the invention relates to compound of formula I wherein R is ethyl or methyl; $R_1$ is fluoro; $R_2$ is hydrogen or fluoro; $R_3$ is hydrogen, fluoro, ethoxy or hydroxy; $R_4$ is hydrogen or fluoro; and $R_5$ is chloro, fluoro or methyl; pharmaceutically acceptable salts thereof.

Further preferred are said compounds wherein R is methyl or ethyl; $R_1$ is fluoro; $R_2$-$R_4$ are hydrogen or fluoro; and $R_5$ is chloro or fluoro; pharmaceutically acceptable salts thereof.

A further embodiment of the invention relates to the compounds of formula I wherein R is methyl or ethyl; $R_1$ is fluoro; $R_2$ is fluoro; $R_3$ is hydrogen, ethoxy or hydroxy; $R_4$ is fluoro; and $R_5$ is fluoro; pharmaceutically acceptable salts thereof.

Another preferred embodiment of the invention relates to the compounds of formula I wherein R is methyl; $R_1$ is fluoro; $R_2$ is hydrogen; $R_3$ is hydrogen or fluoro; $R_4$ is hydrogen; and $R_5$ is chloro; pharmaceutically acceptable salts thereof.

Particular embodiments of the invention relate to compounds of formula I

(a) wherein R is methyl; $R_1$ is fluoro; $R_2$ is hydrogen; $R_3$ is hydrogen; $R_4$ is hydrogen; and $R_5$ is chloro; pharmaceutically acceptable salts thereof.

(b) wherein R is methyl; $R_1$ is fluoro; $R_2$ is hydrogen; $R_3$ is fluoro; $R_4$ is hydrogen; and $R_5$ is chloro; pharmaceutically acceptable salts thereof.

(c) wherein R is ethyl; $R_1$ is fluoro; $R_2$ is fluoro; $R_3$ is hydrogen; $R_4$ is fluoro; and $R_5$ is fluoro; pharmaceutically acceptable salts thereof. and

(d) wherein R is ethyl; $R_1$ is chloro; $R_2$ is hydrogen; $R_3$ is chloro; $R_4$ is hydrogen; and $R_5$ is methyl; pharmaceutically acceptable salts thereof.

The general definitions used herein have the following meaning within the scope of the present invention.

Pharmaceutically acceptable salts represent metal salts, such as alkaline metal salts, e.g. sodium, potassium, magnesium or calcium salts, as well as ammonium salts, which are formed e.g. with ammonia and mono- or di-alkylamines, such as diethylammonium salts, and with amino acids, such as arginine and histidine salts.

The compound 5-methyl-2-(2'-chloro-6'-fluoro-anilino)-phenyl acetic acid, as well as its pharmaceutically acceptable salts, is an especially useful COX-2 inhibitor for use in the present invention.

Furthermore, the present invention relates to the preparation of a medicament for the treatment of pre-malignant colon lesions or a colon cancer or other malignancy in a mammal, which comprises treating the mammal concurrently with a combination of (a) a selective COX-2 inhibitor of the formula (I) wherein R is methyl or ethyl; $R_1$ is chloro or fluoro; $R_2$ is hydrogen or fluoro; $R_3$ is hydrogen, fluoro, chloro, methyl, ethyl, methoxy, ethoxy or hydroxy; $R_4$ is hydrogen or fluoro; and $R_5$ is chloro, fluoro, trifluoromethyl or methyl; pharmaceutically acceptable salts or solvates thereof; and (b) an epothilone.

Moreover, the present invention relates to a combination which comprises (a) a selective COX-2 inhibitor of the formula (I) wherein R is methyl or ethyl; $R_1$ is chloro or fluoro; $R_2$ is hydrogen or fluoro; $R_3$ is hydrogen, fluoro, chloro, methyl, ethyl, methoxy, ethoxy or hydroxy; $R_4$ is hydrogen or fluoro; and $R_5$ is chloro, fluoro, trifluoromethyl or methyl; pharmaceutically acceptable salts or solvates thereof; and (b) an epothilone, in which the active ingredients (a) and (b) are present in each case in free form or in the form of a pharmaceutically acceptable salt and optionally at least one pharmaceutically acceptable carrier; for simultaneous, separate or sequential use.

Preferred combinations are in particular those wherein

- the selective COX-2 inhibitor of the formula (I) is 5-methyl-2-(2'-chloro-6'-fluoro-anilino)-phenyl acetic acid or a pharmaceutically acceptable salt thereof,

- the combination partner (b) is an epothilone, in particular epothilone B,

The combinations disclosed herein are suitable in particular for the preparation of a medicament for the treatment of a proliferative disease and for use in the preparation of a medicament for the treatment of pre-malignant colon lesions or colon cancer.

According to the present invention, a patient is treated, e.g., concurrently with a COX-2 inhibitor and an epothilone, in order to prevent or treat pre-malignant colon lesions, such as polyps, or colon cancer, or another malignancy each according to a dosage schedule that is appropriate for the individual agent. For example, the COX-2 inhibitor may be administered once or more daily and an epothilone once daily, on alternate days or on some other schedule - as is appropriate for the epothilone when used without the COX-2 inhibitor.

Epothilones are known and clinically used for the treatment of cancer. Such compounds include epothilones, such as epothilones A, B, C and D, as well as analogs and derivatives thereof, for example the compounds disclosed in WO 99/02514, particularly [1S-[1R, 3R(E), 7R, 10S, 11R, 12R, 16S]]-7,11-dihydroxy-8,8,10,12,16-pentamethyl-3-[1-methyl-2-(2-methyl-4-thiazolyl)ethenyl]-4-aza-17-bicyclo[14.1.0]-heptadecane-5,9-dione (example 3).

Epothilones that can be used in the present invention are described by formula (II),

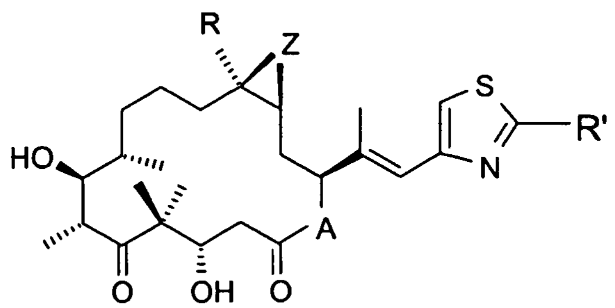

(II)

wherein A represents O or $NR_N$, wherein $R_N$ is hydrogen or lower alkyl, R is hydrogen or lower alkyl, R' is methyl, methoxy, ethoxy, amino, methylamino, dimethylamino or methylthio, and Z is O or a bond.

Unless stated otherwise, in the present disclosure organic radicals and compounds designated "lower" contain not more than 7, preferably not more than 4, carbon atoms.

A compound of formula II wherein A represents O, R is hydrogen, R' is methyl and Z is O is known as epothilone A; a compound of formula II wherein A represents O, R is methyl, R' is methyl and Z is O is known as epothilone B; a compound of formula II wherein A represents O, R is hydrogen, R' is methyl and Z is a bond is known as epothilone C; a compound of formula II wherein A represents O, R is methyl, R' is methyl and Z is a bond is known as epothilone D.

Epothilone derivatives of formula II wherein A represents O or $NR_N$, wherein $R_N$ is hydrogen or lower alkyl, R is hydrogen or lower alkyl, R' is methyl and Z is O or a bond, and methods for the preparation of such epothilone

derivatives are in particular generically and specifically disclosed in the patents and patent applications WO 93/10121, US 6,194,181, WO 98/25929, WO 98/08849, WO 99/43653, WO 98/22461 and WO 00/31247 in each case in particular in the compound claims and the final products of the working examples. Comprised are likewise the corresponding stereoisomers as well as the corresponding crystal modifications, e.g. solvates and polymorphs, which are disclosed therein. Epothilone derivatives of formula II, especially epothilone B, can be administered as part of pharmaceutical compositions which are disclosed in WO 99/39694.

Epothilone derivatives of formula II wherein A represents O or $NR_N$, wherein $R_N$ is hydrogen or lower alkyl, R is hydrogen or lower alkyl, R' is methoxy, ethoxy, amino, methylamino, dimethylamino or methylthio, and Z is O or a bond, and methods for the preparation and administration of such epothilone derivatives are in particular generically and specifically disclosed in the patent application WO99/67252. Comprised are likewise the corresponding stereoisomers as well as the corresponding crystal modifications, e.g. solvates and polymorphs, which are disclosed therein.

The transformation of epothilone B to the corresponding lactam is disclosed in Scheme 21 (page 31, 32) and Example 3 of WO 99/02514 (pages 48 - 50). The transformation of a compound of formula II which is different from epothilone B into the corresponding lactam can be accomplished analogously. Corresponding epothilone derivatives of formula II wherein $R_N$ is lower alkyl can be prepared by methods known in the art such as a reductive alkylation reaction starting from the epothilone derivative wherein $R_N$ is hydrogen.

It is known that MIA compounds such as paclitaxel, discodermolide, colchicine and vinblastine increase levels of prostaglandin E2 through upregulation of COX-2. Without being bound to any particular hypothesis, it is postulated that this effect may partially counteract the antiproliferative effects of epthilones. Thus, enhancement of the anti-tumor activity of the epothilone by inhibition of COX-2 may be basis for the improved effect observed when, according to the present invention, a COX-2 inhibitor is added to a cancer treatment regimen with an epothilone. As an added benefit, the COX-2 inhibitor may help manage cancer-related pain and inflammation.

In one aspect, the present invention relates to the preparation of a medicament for the treatment of pre-malignant colon lesions (e.g. polyps), and colon cancers and other malignancies in a mammal, preferably a human patient, which comprises treating the patient concurrently with a combination of (a) a COX-2 inhibitor of U.S. Patent No. 6,291,523 and (b) an epothilone.

In addition to the preparation of a medicament for the treatment of pre-malignant colon lesions (e.g. polyps) and colon cancer, the inventive combination therapy has utility for the treatment of "other malignancies", which is hereby defined as a malignancy that is suseptible to treatment with an epothilone, for example, breast cancer, lung cancer, ovarian cancer, lymphoma, head and neck cancer and cancer of the esophagus, stomach, bladder, prostrate, uterus and cervix.

Most preferably, the present invention relates to the preparation of a medicament for the treatment of pre-malignant colon lesions, colon cancer or another malignancy in a human patient, which comprises treating the patient concurrently with a combination of (a) a COX-2 inhinitor selected from the group consisting of 5-methyl-2-(2'-chloro-6'-fluoro-anilino)-phenyl acetic acid, or a pharmaceutically acceptable salt thereof, and (b) an epothilone. Especially, the epothilone compound is epothilone B. In a specific embodiment, the inventive use is a preparation of a medicament for the treatment of colon cancer. In another embodiment, the inventive use is for the preparation of a medicament for the treatment of other malignancies as described above.

Preferably, treatment using a COX-2 inhibitor within one of the preferred classes disclosed in U.S. Patent No. 6,291,523 is combined with treatment using an epothilone. Preferably, the epothilone is epothilone B.

The structure of the active agents identified by code nos., generic or trade names may be taken from the actual edition of the standard compendium "The Merck Index" or from databases, e.g. Patents International (e.g. IMS World Publications).

The present invention further relates to "a combined preparation", which, as used herein, defines especially a "kit of parts" in the sense that the combination partners (a) and (b) as defined above can be dosed independently or by use of different fixed combinations with distinguished amounts of the combination partners (a) and (b), i.e., simultaneously or at different time points. The parts of the kit of parts can then, e.g., be administered simultaneously or chronologically staggered, that is at different time points and with equal or different time intervals for any part of the kit of parts. The ratio of the total amounts of the combination partner (a) to the combination partner (b) to be administered in the combined preparation can be varied, e.g. in order to cope with the needs of a patient sub-population to be treated or the needs of the single patient based on the severity of the diarrhea that the patient experiences.

The present invention especially relates to a combined preparation, in particular wherein the unit dosage forms are for oral administration, which comprises (a) one or more unit dosage forms of a COX-2 inhibitor and (b) one or more unit dosage forms of an epothilone. The present invention especially relates to a combined preparation, which comprises (a) one or more unit dosage forms of a COX-2 inhinitor selected from the group consisting of 5-methyl-2-(2'-chloro-6'-fluoro-anilino)-phenyl acetic acid, or a pharmaceutically acceptable salt thereof, and (b) one or more unit dosage forms of an epothilone. Preferably, the epothilone is epothilone B.

The combination partner (a) or (b) or a pharmaceutically acceptable salt thereof may also be used in form of

a hydrate or other solvate.

A combination which comprises which comprises (a) a selective cyclooxygenase-2 inhibitor of the formula (I) wherein R is methyl or ethyl; $R_1$ is chloro or fluoro; $R_2$ is hydrogen or fluoro; $R_3$ is hydrogen, fluoro, chloro, methyl, ethyl, methoxy, ethoxy or hydroxy; $R_4$ is hydrogen or fluoro; and $R_5$ is chloro, fluoro, trifluoromethyl or methyl; pharmaceutically acceptable salts or solvates thereof; and (b) an epothilone, in which the active ingredients (a) and (b) are present in each case in free form or in the form of a pharmaceutically acceptable salt and optionally at least one pharmaceutically acceptable carrier, will be referred to hereinafter as a COMBINATION OF THE INVENTION.

Suitable clinical studies are in particular randomized, double-blind, placebo-controlled, parallel studies in cancer patients with late stage disease, e.g. colon cancer. Such studies are, in particular, suitable to compare the effects of a monotherapy using the active ingredients and a therapy using a COMBINATION OF THE INVENTION, and to prove in particular the synergism of the active ingredients of the COMBINATIONS OF THE INVENTION. The primary endpoints in such studies can be the effect on pain scores, analgesic use, performance status, Quality of Life scores or time to progression of the disease. The radiologic evaluation of tumors in regular time periods, e.g. every 4, 6, 8 or 10 weeks, is a suitable approach to determine the effect of the COMBINATION OF THE INVENTION. In a suitable study design, patients are, for example, randomized in a double-blind fashion receiving a fixed dosage of a COX-2 inhibitor or a corresponding placebo in addition to treatment cycles employing a compound of formula II, e.g. epothilone B, wherein each cycle consists of 0.5, 1.0, 1.5, 2.0 or 2.5 $mg/m^2$ epothilone B administered as a 5 minute bolus injection once a week for three weeks followed by one week of rest. Alternatively, the compound of formula II can be administered once every three weeks. The minimum duration of such a study should be about 8 weeks.

It is one objective of this invention to provide a pharmaceutical composition comprising a quantity, which is therapeutically effective pre-malignant colon lesions or colon cancer or other malignancy comprising the COMBINATION OF THE INVENTION. In this composition, the combination partners (a) and (b) can be administered together, one after the other or separately in one combined unit dosage form or in two separate unit dosage forms. The unit dosage form may also be a fixed combination.

When the combination partners employed in the COMBINATION OF THE INVENTION are applied in the form as marketed as single drugs, their dosage and mode of administration can take place in accordance with the information provided on the package insert of the respective marketed drug in order to result in the beneficial effect described herein, if not mentioned herein otherwise.

The effective dosage of each of the combination partners employed in the COMBINATION OF THE INVENTION may vary depending on the particular compound or pharmaceutical composition employed, the mode of administration, the condition being treated, the severity of the condition being treated. Thus, the dosage regimen the COMBINATION OF THE INVENTION is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites.

In the instance where the COX-2 inhibitor is 5-methyl-2-(2'-chloro-6'-fluoro-anilino)-phenyl acetic acid, or a pharmaceutically acceptable salt thereof, an appropriate dose is in the range from 100 to 1500 mg of 5-methyl-2-(2'-chloro-6'-fluoro-anilino)-phenyl acetic acid daily, for example, 200-1000 mg/day, such as 200, 400, 500, 600, 800, 900 or 1000 mg/day, administered in one or two doses daily. Preferably, 5-methyl-2-(2'-chloro-6'-fluoro-anilino)-phenyl acetic acid, or a pharmaceutically acceptable salt thereof is administered as an oral pharmaceutal formulation in the form of a tablet, capsule or syrup.

As used herein, the expression "week" means seven consecutive days. Thus, a three week period is twenty-one consecutive days starting on any day of the calendar week. The day that the first dose is given is considered to be the first day of the week. Any discussion using calendar weeks is intended to be for illustrative purposes only.

Preferably, (R)-4-(4-((1-phenylethyl)amino)-7H-pyrrolo(2,3-d)pyrimidin-6-yl)-phenol, or a pharmaceutically acceptable salt therof, or a pharmaceutically acceptable salt thereof, is administered as an oral pharmaceutal formulation in the form of a tablet, capsule or syrup.

Epothilone B is preferably administered in a dose which is calculated according to the formula (III)

$$\text{single dose (mg/m2)} = (0.1 \text{ to } y) \times N \qquad \text{(III)}$$

wherein N is the number of weeks between treatments and y is 6, wherein epothilone B is administered in more than one treatment cycle after an interval of one week to six weeks after the preceding treatment.

In one preferred embodiment of the invention, epothilone B is administered weekly in a dose that is between

about 0.1 to 6 $mg/m^2$, preferably between 0.1 and 3 $mg/m^2$, e.g. 2.5 or 3.0 $mg/m^2$, for three weeks after an interval of one to six weeks, especially an interval of one week, after the preceding treatment. In another embodiment of the invention said epothilone B is preferably administered to a human every 18 to 24 days in a dose that is between about 0.3 and 12 $mg/m^2$.

Moreover, the present invention pertains to the use of a combination which comprises (a) a selective cyclooxygenase-2 inhibitor, in particular a selective cyclooxygenase-2 inhibitor of the formula (I) wherein the radicals and symbols have the meanings as provided above or a pharmaceutically acceptable prodrug ester thereof, and (b) an epothilone, in which the active ingredients (a) and (b) are present in each case in free form or in the form of a pharmaceutically acceptable salt and optionally at least one pharmaceutically acceptable carrier; for the preparation of a medicament for the treatment of a proliferative disease, in particular for the prevention or treatment of pre-malignant colon lesions or colon cancer.

## Claims

1. A combination which comprises (a) a selective cyclooxygenase-2 inhibitor of the formula (I)

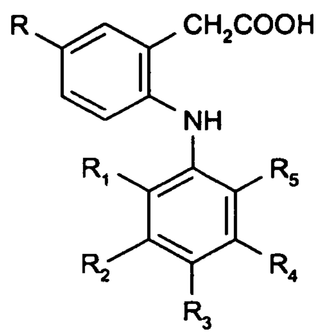

(I)

wherein R is methyl or ethyl;
$R_1$ is chloro or fluoro;
$R_2$ is hydrogen or fluoro;
$R_3$ is hydrogen, fluoro, chloro, methyl, ethyl, methoxy, ethoxy or hydroxy;
$R_4$ is hydrogen or fluoro; and
$R_5$ is chloro, fluoro, trifluoromethyl or methyl;
pharmaceutically acceptable salts or solvates thereof; and
(b) an epothilone,
in which the active ingredients (a) and (b) are present in each case in free form or in the form of a pharmaceutically acceptable salt and optionally at least one pharmaceutically acceptable carrier; for simultaneous, separate or sequential use.

2. Combination according to claim 1 wherein the selective cyclooxygenase-2 inhibitor of the formula (I) is 5-methyl-2-(2'-chloro-6'-fluoro-anilino)-phenyl acetic acid or a pharmaceutically acceptable salt thereof.

3. Combination according to any one of claims 1 or 2 for use in the treatment of a proliferative disease.

4. Combination according to any one of claims 1 or 2 for use in the prevention or treatment of pre-malignant colon lesions or colon cancer.

5. Use of a combination according to any one of claims 1 or 2 for the preparation of a medicament for the treatment of a proliferative disease.

6. Use of a combination according to any one of claims 1 or 2 for the preparation of a medicament for the prevention or treatment of pre-malignant colon lesions or colon cancer.

7. Use of a combination according to any one of claims 1 or 2 for the preparation of a medicament for the treatment

of cancer of the prostate.

**8.** Use of a selective cyclooxygenase-2 inhibitor of the formula (I)

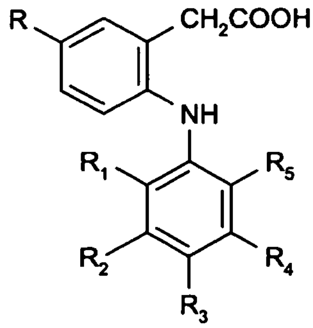

(I)

wherein R is methyl or ethyl;
$R_1$ is chloro or fluoro;
$R_2$ is hydrogen or fluoro;
$R_3$ is hydrogen, fluoro, chloro, methyl, ethyl, methoxy, ethoxy or hydroxy;
$R_4$ is hydrogen or fluoro; and
$R_5$ is chloro, fluoro, trifluoromethyl or methyl;
pharmaceutically acceptable salts or solvates thereof;
and
in combination with (b) an epothilone,
for the preparation of a medicament for the treatment of a proliferative disease.

**9.** A commercial package comprising (a) a selective cyclooxygenase-2 inhibitor of the formula (I)

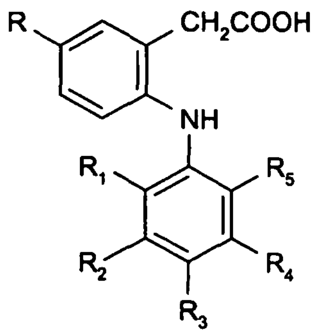

(I)

wherein R is methyl or ethyl;
$R_1$ is chloro or fluoro;
$R_2$ is hydrogen or fluoro;
$R_3$ is hydrogen, fluoro, chloro, methyl, ethyl, methoxy, ethoxy or hydroxy;
$R_4$ is hydrogen or fluoro; and
$R_5$ is chloro, fluoro, trifluoromethyl or methyl;
pharmaceutically acceptable salts or solvates thereof;
and (b) an epothilone, together with instructions for simultaneous, separate or sequential use thereof in the treatment of a proliferative disease.

**10.** A combined preparation which comprises (a) one or more unit dosage forms of a COX-2 inhibitor selected from the group consisting of 5-methyl-2-(2'-chloro-6'-fluoro-anilino)-phenyl acetic acid, or a pharmaceutically acceptable salt thereof, and (b) an epothilone.

**11.** The combined preparation according to claim 10 wherein the microtubule interfering agent is epothilone B.

**12.** A combined preparation according to claim 10 to 11 wherein the unit dosage forms are for oral administration.

## Patentansprüche

**1.** Kombination, umfassend

(a) einen selektiven Cyclooxygenase-2-Inhibitor der Formel (I)

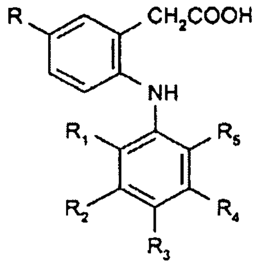

(I)

worin
R für Methyl oder Ethyl steht,
$R_1$ für Chlor oder Fluor steht,
$R_2$ für Wasserstoff oder Fluor steht,
$R_3$ für Wasserstoff, Fluor, Chlor, Methyl, Ethyl, Methoxy, Ethoxy oder Hydroxy steht,
$R_4$ für Wasserstoff oder Fluor steht, und
$R_5$ für Chlor, Fluor, Trifluormethyl oder Methyl steht,
oder pharmazeutisch akzeptable Salze oder Solvate hiervon, und
(b) ein Epothilon,
worin die Wirkstoffe (a) und (b) jeweils in freier Form oder in Form eines pharmazeutisch akzeptablen Salzes vorhanden sind, und
(c) optional wenigstens einen pharmazeutisch akzeptablen Träger,

für eine simultane, separate oder sequentielle Verwendung.

**2.** Kombination nach Anspruch 1, worin der selektive Cyclooxygenase-2-Inhibitor der Formel (I) 5-Methyl-2-(2'-chlor-6'-fluoranilino)-phenylessigsäure oder ein pharmazeutisch akzeptables Salz hiervon ist.

**3.** Kombination nach einem der Ansprüche 1 oder 2 zur Verwendung für die Behandlung einer proliferativen Krankheit.

**4.** Kombination nach einem der Ansprüche 1 oder 2 zur Verwendung für die Prävention oder Behandlung von prämalignen Colonläsionen oder Colonkrebs.

**5.** Verwendung einer Kombination nach einem der Ansprüche 1 oder 2 zur Herstellung eines Arzneimittels für die Behandlung einer proliferativen Krankheit.

**6.** Verwendung einer Kombination nach einem der Ansprüche 1 oder 2 zur Herstellung eines Arzneimittels für die Prävention oder Behandlung von prämalignen Colonläsionen oder Colonkrebs.

**7.** Verwendung einer Kombination nach einem der Ansprüche 1 oder 2 zur Herstellung eines Arzneimittels für die Behandlung von Prostatakrebs.

**8.** Verwendung einer Kombination von

(a) einem selektiven Cyclooxygenase-2-Inhibitor der Formel (I)

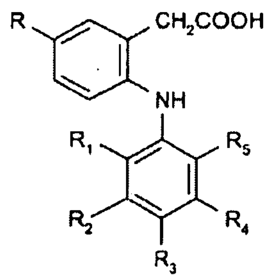

(I)

worin
R für Methyl oder Ethyl steht,
$R_1$ für Chlor oder Fluor steht,
$R_2$ für Wasserstoff oder Fluor steht,
$R_3$ für Wasserstoff, Fluor, Chlor, Methyl, Ethyl, Methoxy, Ethoxy oder Hydroxy steht,
$R_4$ für Wasserstoff oder Fluor steht, und
$R_5$ für Chlor, Fluor, Trifluormethyl oder Methyl steht,
oder einem pharmazeutisch akzeptablen Salz oder Solvat hiervon, mit
(b) einem Epothilon,

zur Herstellung eines Arzneimittels für die Behandlung einer proliferativen Krankheit.

**9.** Handelspackung, umfassend

(a) einen selektiven Cyclooxygenase-2-Inhibitor der Formel (I)

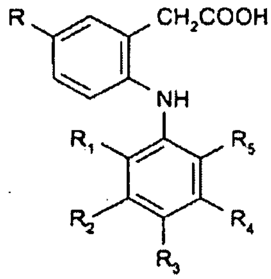

(I)

worin
R für Methyl oder Ethyl steht,
$R_1$ für Chlor oder Fluor steht,
$R_2$ für Wasserstoff oder Fluor steht,
$R_3$ für Wasserstoff, Fluor, Chlor, Methyl, Ethyl, Methoxy, Ethoxy oder Hydroxy steht,
$R_4$ für Wasserstoff oder Fluor steht, und
$R_5$ für Chlor, Fluor, Trifluormethyl oder Methyl steht,
oder ein pharmazeutisch akzeptables Salz oder Solvat hiervon, und
(b) ein Epothilon,

zusammen mit Instruktionen zur simultanen, separaten oder sequentiellen Verwendung hiervon für die Behandlung einer proliferativen Krankheit.

**10.** Kombinationspräparat, umfassend (a) ein oder mehr Einheitsdosierungsformen eines COX-2-Inhibitors, der ausgewählt ist aus der Gruppe, die besteht aus 5-Methyl-2-(2'-chlor-6'-fluoranilino)-phenylessigsäure oder einem pharmazeutisch akzeptablen Salz hiervon, und (b) einem Epothilon.

**11.** Kombinationspräparat nach Anspruch 10, worin das Mikrotubuli interferierende Mittel Epothilon B ist.

**12.** Kombinationspräparat nach Anspruch 10 bis 11, worin die Einheitsdosierungsformen für eine orale Verabreichung sind.

## Revendications

**1.** Combinaison qui comprend (a) un inhibiteur sélectif de la - cyclooxygénase-2 de formule (I) :

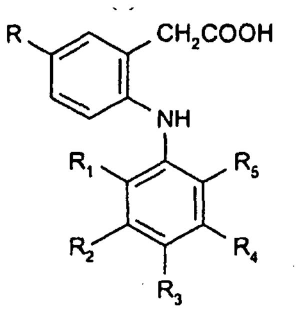

(I)

dans laquelle R est le groupe méthyle ou éthyle ;
$R_1$ est le groupe chloro ou fluoro ;
$R_2$ est un atome d'hydrogène ou le groupe fluoro ;
$R_3$ est un atome d'hydrogène ou le groupe fluoro, chloro, méthyle, éthyle, méthoxy, éthoxy ou hydroxy ;
$R_4$ est un atome d'hydrogène ou le groupe fluoro ; et
$R_5$ est le groupe chloro, fluoro, trifluorométhyle ou méthyle ;
ses sels ou produits de solvatation acceptables d'un point de vue pharmaceutique ; et
(b) une épothilone,
où les principes actifs (a) et (b) sont présents dans chaque cas sous forme libre ou sous forme d'un sel acceptable d'un point de vue pharmaceutique, et en option au moins un excipient acceptable d'un point de vue pharmaceutique ; pour utilisation simultanée, distincte ou séquentielle.

**2.** Combinaison selon la revendication 1, dans laquelle l'inhibiteur sélectif de la cyclooxygénase-2 de formule (I) est l'acide 5-méthyl-2-(2'-chloro-6'-fluoro-anilino)-phénylacétique ou un sel acceptable d'un point de vue pharmaceutique de ce dernier.

**3.** Combinaison selon l'une quelconque des revendications 1 ou 2 pour utilisation dans le traitement d'une maladie proliférative.

**4.** Combinaison selon l'une quelconque des revendications 1 ou 2 pour utilisation dans la prévention ou le traitement de lésions prémalignes du côlon ou du cancer du côlon.

**5.** Utilisation d'une combinaison selon l'une quelconque des revendications 1 ou 2 pour la préparation d'un médicament destiné au traitement d'une maladie proliférative.

**6.** Utilisation d'une combinaison selon l'une quelconque des revendications 1 ou 2 pour la préparation d'un médicament destiné à la prévention ou au traitement de lésions prémalignes du côlon ou du cancer du côlon.

**7.** Utilisation d'une combinaison selon l'une quelconque des revendications 1 ou 2 pour la préparation d'un médicament destiné au traitement du cancer de la prostate.

**8.** Utilisation d'un inhibiteur sélectif de la cyclooxygénase-2 de formule (I) :

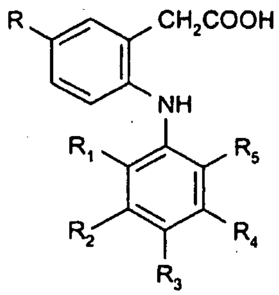

(I)

dans laquelle R est le groupe méthyle ou éthyle ;
$R_1$ est le groupe chloro ou fluoro ;
$R_2$ est un atome d'hydrogène ou le groupe fluoro ;
$R_3$ est un atome d'hydrogène ou le groupe fluoro, chloro, méthyle, éthyle, méthoxy, éthoxy ou hydroxy ;
$R_4$ est un atome d'hydrogène ou le groupe fluoro ; et
$R_5$ est le groupe chloro, fluoro, trifluorométhyle ou méthyle ;
de ses sels ou produits de solvatation acceptables d'un point de vue pharmaceutique ;
en combinaison avec (b) une épothilone,
pour la préparation d'un médicament destiné au traitement d'une maladie proliférative.

**9.** Conditionnement du commerce comprenant (a) un inhibiteur sélectif de la cyclooxygénase-2 de formule (I) :

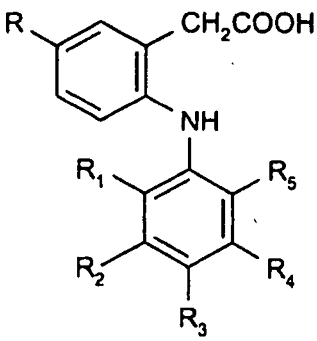

(I)

dans laquelle R est le groupe méthyle ou éthyle ;
$R_1$ est le groupe chloro ou fluoro ;
$R_2$ est un atome d'hydrogène ou le groupe fluoro ;
$R_3$ est un atome d'hydrogène ou le groupe fluoro, chloro, méthyle, éthyle, méthoxy, éthoxy ou hydroxy ;
$R_4$ est un atome d'hydrogène ou le groupe fluoro ; et
$R_5$ est le groupe chloro, fluoro, trifluorométhyle ou méthyle ;
des sels ou produits de solvatation acceptables d'un point de vue pharmaceutique de ce composé ;
et (b) une épothilone, en même temps que des instructions pour leur utilisation simultanée, distincte ou séquentielle, dans le traitement d'une maladie proliférative.

**10.** Préparation en combinaison qui comprend (a) une ou plusieurs formes posologiques unitaires d'un inhibiteur de la COX-2 choisi dans le groupe consistant en l'acide 5-méthyl-2-(2'-chloro-6'-fluoro-anilino)-phénylacétique ou un sel acceptable d'un point de vue pharmaceutique de ce dernier, et (b) une épothilone.

**11.** Préparation en combinaison selon la revendication 10, dans laquelle l'agent interférant avec les microtubules est l'épothilone B.

**12.** Préparation en combinaison selon les revendications 10 à 11, où les formes posologiques unitaires sont destinées à une administration orale.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9911605 A1
- WO 0160365 A1
- EP 0927555 A
- US 6034256 A
- US 6291523 B
- WO 9902514 A
- WO 9310121 A
- US 6194181 B
- WO 9825929 A
- WO 9808849 A
- WO 9943653 A
- WO 9822461 A
- WO 0031247 A
- WO 9939694 A
- WO 9967252 A